(19) Europäisches Patentamt
European Patent Office
Office européen des brevets

(11) Veröffentlichungsnummer : **0 056 125**
**B1**

(12) # EUROPÄISCHE PATENTSCHRIFT

(45) Veröffentlichungstag der Patentschrift : **05.09.84**

(51) Int. Cl.³ : **C 07 D249/08**, C 07 D233/60, A 01 N 43/64, A 01 N 43/50

(21) Anmeldenummer : **81110350.6**

(22) Anmeldetag : **11.12.81**

(54) **Keten-O,N-acetale, Verfahren zu ihrer Herstellung, diese enthaltende Fungizide und ihre Verwendung zur Bekämpfung von Pilzen.**

(30) Priorität : **08.01.81 DE 3100261**

(43) Veröffentlichungstag der Anmeldung : **21.07.82 Patentblatt 82/29**

(45) Bekanntmachung des Hinweises auf die Patenterteilung : **05.09.84 Patentblatt 84/36**

(84) Benannte Vertragsstaaten : **AT BE CH DE FR GB IT LI LU NL SE**

(56) Entgegenhaltungen :
**EP-A- 0 001 414**
**EP-A- 0 011 191**
**EP-A- 0 015 387**
**EP-A- 0 023 614**
**EP-A- 0 023 653**
**US-A- 4 182 862**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Reuther, Wolfgang, Dr.**
**Am Pferchelhang 16**
**D-6900 Heidelberg (DE)**
Erfinder : **Ruland, Alfred, Dr.**
**Schriesheimer Strasse 11**
**D-6945 Hirschberg (DE)**
Erfinder : **Pommer, Ernst-Heinrich, Dr.**
**Berliner Platz 7**
**D-6703 Limburgerhof (DE)**

EP 0 056 125 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Keten-O,N-acetale, ein Verfahren zu ihrer Herstellung und ihre Verwendung als Fungizide, sowie diese enthaltende Fungizide.

Es ist bekannt, 1-(2'-[2,4-Dichlorphenyl]-2'-[2-propenyloxyl]-äthyl-1H-imidazol (GB-A-1 318 590) und das Zink-äthylen-bisdithiocarbamat (Chemical Week, July 26, 1972, Seite 41) als Fungizide zu verwenden. Ihre Wirkung ist jedoch unbefriedigend.

Es ist ferner bekannt, Triazoyl-O,N-acetale, z. B. 1-2,4-(Dichlorphenoxy)-3,3-dimethyl-1-(1,2,4-triazol-1-yl)-butan-2-ol, als Fungizide zu verwenden (EP-A-1414). Weiterhin ist bekannt, 1-Vinyltriazolderivate, z. B. das 1-(2,4-Dichlorphenyl)-2-(1,2,4-Triazol-1-yl)-4-Methyl-hept-2-en-1-ol, als Fungizide zu verwenden (EP-A-15387). Hierdurch werden die neuen Verbindungen nicht nahegelegt.

Es wurden nun gefunden, daß Keten-O,N-acetale der allgemeinen Formel I

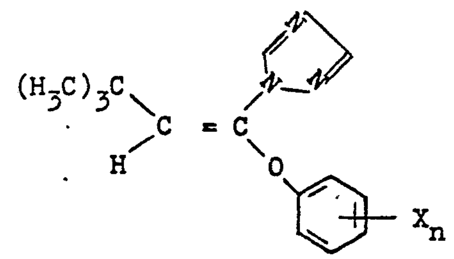

(I)

in welcher X Halogen, Alkyl mit 1 bis 4 C-Atomen, oder Phenyl bedeuten, und n 1 bis 3 bedeutet, eine überraschend gute fungizide Wirkung haben.

Es wurde ferner gefunden, daß sich die Verbindungen der allgemeinen Formel I sehr leicht und in guten Ausbeuten durch Zersetzung aus den Sulfonaten der Formel II

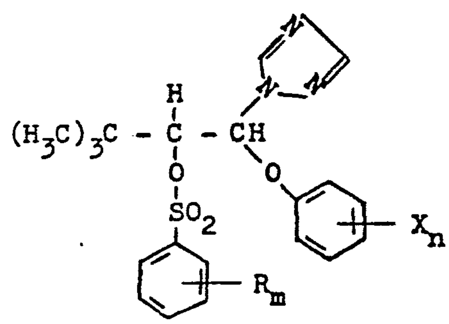

(II)

herstellen lassen, in der X und n die im Anspruch 1 angegebene Bedeutung haben und R Wasserstoff, Halogen, Alkyl mit 1 bis 4 C-Atomen oder Alkoxy mit 1 bis 4 C-Atomen und m 1 bis 3 bedeutet.

Die Zersetzung wird in Anwesenheit eines Lösungsmittels z. B. Acetonitril, Dimethylformamid, Dimethylsulfoxid, Hexamethylphosphorsäuretriamid, vorzugsweise Dimethylsulfoxid und von basischen Katalysatoren wie Alkalihydroxiden, -sulfiden und -carbonaten, vorzugsweise Natriumsulfid, bei Temperaturen zwischen 10 und 100 °C durchgeführt. Die für die Zersetzung benötigten Sulfonate der Formel II lassen sich aus den entsprechenden Metallakoholaten III, die nach bekannten Verfahren hergestellt werden können und in der Literatur beschrieben sind, durch Umsetzung mit den Sulfonsäurechloriden IV gewinnen.

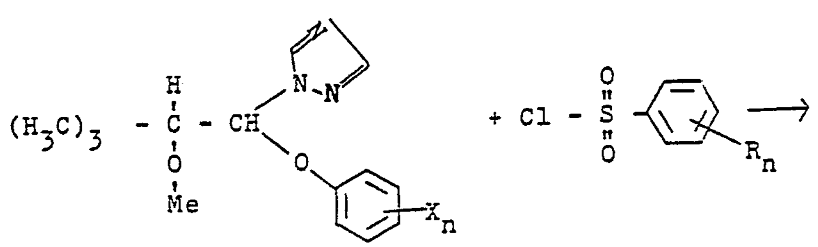

(II)

(III) (IV)

Die Verbindungen der Formel I können als E- oder Z-Isomere vorliegen, je nach Anordnung der Gruppen an den C-Atomen der Doppelbindung. Sowohl die reinen Isomeren als auch ihre Gemische werden von der Erfindung umfaßt.

Herstellungsbeispiel

a) Tosylat des 1-(1,2,4-Triazol-1-yl)-1-(4-chlorphenoxy)-3,3-dimethyl-butan-2-ols

44,6 g (0,15 mol) 1-(1,2,4-Triazol-1-yl)-1-(4-chlorphenoxy)-3,3-dimethyl-butan-2-ol (als Diastereomerengemisch) werden in 500 ml absolutem Tetrahydrofuran (THF) gelöst und unter Rühren bei Raumtemperatur mit 4,5 g Natriumhydrid (~ 80 %ig) (Gew.%) versetzt. Danach wird 6 bis 8 Stunden bei 40 bis 50 °C gerührt und nach dem Abkühlen 28,5 g (0,15 mol) p-Toluolsulfochlorid, gelöst in möglichst wenig THF, zugetropft. Man rührt weitere 5 bis 6 Stunden bei Raumtemperatur, hydrolysiert dann mit Wasser, extrahiert mehrfach mit je 500 ml Methylenchlorid, trocknet die organische Phase über Natriumsulfat und destilliert das Lösungsmittel im Vakuum ab. Der verbleibende Rückstand wird aus Essigester fraktioniert kristallisiert, wobei 23,5 g (35 % d. Th. bezogen auf Diastereomerengemisch) an Tosylat als Diastereomerengemisch vom Schmp. 155 bis 157 °C erhalten werden.

b) 1-(1,2,4-Triazol-1-yl)-1-(4-chlorphenoxy)-3,3-dimethyl)-buten-1

22,4 g (0,05 mol) des oben erhaltenen Tosylats werden in 250 ml Dimethylsulfoxid gelöst und mit 30 g Natriumsulfid versetzt. Man rührt 1 Stunde bei Raumtemperatur (20 °C), versetzt danach das Reaktionsgemisch mit 500 ml Diethyl-Ether, trocknet die organische Phase über Natriumsulfat und destilliert das Lösungsmittel in Vakuum ab. Man erhält 11,8 g (85 % d. Th.) an weitgehend reinem 1-(1,2,4-Triazol-1-yl)-1-(4-chlorphenoxy)-3,3-dimethyl-buten-1 (E, Z-Isomerengemisch) vom Schmp. 75 bis 76 °C (Verbindung Nr. 1).

$C_{14}H_{16}ClN_3O$ (277,754)

ber.: C 60,5 H 5,9 Cl 12,8 N 15,1

gef.: C 60,4 H 6,1 Cl 12,5 N 15,0

Die folgenden Verbindungen werden in entsprechender Weise erhalten.

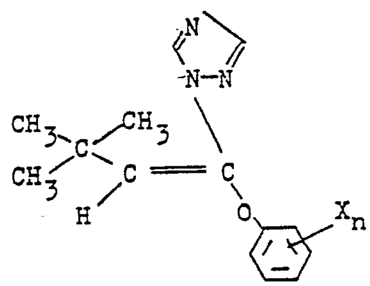

(E-Isomeres)

| Verbindung Nr. | X | n | Isomeres | Physik. Daten 1H-NMR (ppm) in $CDCl_3$ |
|---|---|---|---|---|
| 2 | 4-Cl | 1 | E | Schmp : 75-76 °C<br>δ = 1,0 (s, 9H);<br>5,5 (s, 1H);<br>7,1 (AA'BB', 4H);<br>7,95 (s, 1H);<br>8,2 (s, 1H). |
| 3 | 4-Br | 1 | E | Schmp. 92,5 °C<br>δ = 1,0 (s, 9H);<br>5,55 (s, 1H);<br>7,2 (AA'BB', 4H);<br>8 (s, 1H);<br>8,25 (s, 1H). |
| 4 | 2-Cl | 1 | Z | Öl<br>δ = 1,15 (s, 9H);<br>5,9 (s, 1H);<br>6,75-7,5 (m, 4H);<br>7,89 (s, 1H);<br>8,15 (s, 1H). |

(Fortsetzung)

| Verbindung Nr. | X | n | Isomeres | Physik. Daten 1H-NMR (ppm) in $CDCl_3$ |
|---|---|---|---|---|
| 5 | 4-tert.Butyl | 1 | E-Z-Gemisch | Öl<br>δ = 1,0 (s, 9H) ;<br>5,5 (s, 1H) ;<br>7,1 (AA'BB, 4H) ;<br>7,98 (s, 1H) ;<br>8,2 (s, 1H) E ;<br>δ = 1,15 (s, 9H) ;<br>1,25 (s, 9H) ;<br>5,89 (s, 1H) ;<br>7,08 (AA'BB', 4H) ;<br>7,9 (s, 1H) ;<br>8,1 (s, 1H) Z. |
| 6 | 3,5-Cl | 2 | E | δ = 0,95 (s, 9H) ;<br>5,55 (s, 1H) ;<br>6,9 (m, 3H) ;<br>7,9 (s, 1H) ;<br>8,15 (s, 1H).<br>Öl |
| 7 | 2,4-Cl | 2 | Z | δ = 1,25 (s, 9H) ;<br>5,85 (s, 1H)<br>6,7-7,4 (m, 3H) ;<br>7,85 (s, 1H) ;<br>8,1 (s, 1H). |
| 8 | 2,4-Cl | 2 | E | Öl<br>δ = 0,95 (s, 1H) ;<br>5,3 (s, 1H) ;<br>7,1-7,3 (m, 3H) ;<br>7,9 (s, 1H) ;<br>8,25 (s, 1H). |
| 9 | 4-Phenyl | 1 | | δ = 1,2 (s, 9H) ;<br>5,85 (s, 1H) ;<br>6,8-7,5 (m, 9H) ;<br>7,85 (s, 1H) ;<br>8,05 (s, 1H) ;<br>Schmp. 82-84 °C |
| 10 | 2,4,5-Cl | 3 | E | δ = 0,95 (s, 9H) ;<br>5,4 (s, 1H) ;<br>7,4 (s, 1H) ;<br>7,5 (s, 1H) ;<br>7,95 (s, 1H) ;<br>8,45 (s, 1H) ;<br>Öl |

Die neuen Wirkstoffe haben eine gute fungizide Wirkung. Sie eignen sich besonders zur Bekämpfung von Pilzerkrankungen an verschiedenen Kulturpflanzen, z. B. Erysiphe graminis an Getreide, Uncinula necator an Reben, Podosphaera leucotricha an Äpfeln, Erysiphe cichoracearum an Kürbisgewächsen, Mycosphaerella musicola an Bananen, Phytophthora infestans an Tometen und Kartoffeln, Plasmopara viticola an Reben, Pseudoperonospora humuli an Hopfen.

Einige der Wirkstoffe sind systemisch wirksam ; die zur Bekämpfung phytopathogener Pilze erforderlichen Aufwandmengen liegen zwischen 0,025 und 2 kg Wirkstoff/ha Kulturfläche.

Die neuen Wirkstoffe eignen sich auch zum Schutz von Holz gegen den Befall durch holzzerstörende Pilze, wie Coniophora puteana, Lenzites trabea, Trametes versicolor. Die Aufwandmengen betragen je nach Art des gewünschten Effektes 0,001 bis 5 % (Gewichtsprozent), bezogen auf das Gewicht des zu schützenden Materials, vorzugsweise jedoch zwischen 0,01 und 3 %.

Die Anwendung als Fungizid erfolgt z. B. in Form von direkt versprühbaren Lösungen, Pulvern, Suspensionen oder Dispersionen, Emulsionen, Öldispersionen, Pasten, Stäubemitteln Streumitteln, Granulaten durch Versprühen, Streichen, Tränken, Vernebeln, Verstäuben, Verstreuen oder Gießen. Die Anwendungsformen richten sich ganz nach den Verwendungszwecken ; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Fungizide enthalten z. B. 5 bis 95 % (Gew.%) insbesondere 10 bis 80 % Wirkstoff.

An oberflächenaktiven Stoffen sind zu nennen : Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes Isooctylphenol-, Octylphenol-, Nonylphenol-, Alkylphenolpolyglykoläther. Tributylphenylpolyglykolether, Alkylarylpolyätheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

## Beispiel a

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

## Beispiel b

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoäthanolamid, 2 Gewichtsteilen Calciumsalz der Dodecylbenzolsulfonsäure und 2 Gewichtsteilen des Anlagerungsproduktes von 40 Mol Äthylenoxid an 1 Mol Ricinusöl besteht.

## Beispiel c

20 Gewichtsteile der Verbindung 3 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Äthylenoxid an 1 Mol Isooctylphenol und 5 Gewichtsteilen des Anlagerungsproduktes von 40 Mol Äthylenoxid an 1 Mol Ricinusöl besteht.

## Beispiel d

20 Gewichsteile der Verbindung 1 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht.

Beispiel e

80 Gewichtsteile des Wirkstoffs 1 werden mit 3 Gewichtsteilen des Natriumsalzes der Diisobutylnaphthalin-alphasulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel f

5 Gewichtsteile der Verbindung 1 werden mit 95 Gewichtsteilen feinteiligem Kaolin innig vermischt. Man erhält auf diese Weise ein Stäubemittel, das 5 Gewichtsprozent des Wirkstoffs enthält.

Beispiel g

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 1 werden mit 10 Teilen Natriumsalz eines Phenolsulfonsäureharnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel i

20 Teile des Wirkstoffs 1 werden mit 12 Teilen Calciumsalz der Dodecylbenzosulfonsäure, 8 Teile Fettalkohol-polyglykoläther, 2 Teilen Natriumsalz eines Phenolsulfonsäureharnstoff-formaldehyd-kondensats und 68 Teilen eines paraffinischen Mineralöls innig vermischt. Man erhält eine stabile ölige Dispersion.

Die Fungizide können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise :

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat)
und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat)
und
N,N'-Polypropylen-bis-(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Strukturen, wie
N-Trichlormethylthio-tetrahydrophthalimid,
N-Trichlormethylthio-phthalimid,
2-Heptadecyl-2-imidazolin-acetat,

2,4-Dichlor-6(o-chloranilino)-s-triazin,
O,O-Diäthyl-phthalimidophosphonothioat,
5-Amino-1-(bis-dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol und verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl (2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Versuche erläutern die gute fungizide Wirkung der Wirkstoffe im Vergleich zu bekannten Wirkstoffen.

Als Vergleichsmittel wurden folgende bekannte Verbindungen verwendet :

| | |
|---|---|
| 1-(2'-(2",4"-Dichlorphenyl)-2'-(2"-propenyloxy)-1H-imidazol | (Verbindung A) |
| Zink-äthylen-bis-dithiocarbamat | (Verbindung B) |

## Versuch 1

Wirksamkeit gegen Weizenmehltau

Blätter von in Töpfen gewachsenen Weizenkeimlingen der Sorte « Jubilar » werden mit wäßrigen Emulsionen aus 80 % (Gewichtsprozent) Wirkstoff und 20 % Emulgiermittel (bezogen auf die Trockensubstanz) besprüht und nach dem Antrocknen des Spritzbelages mit Oidien (Sporen) des Weizenmehltaus (Erysiphe graminis var tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 10 Tagen wird das Ausmaß der Mehltauentwicklung ermittelt.

In diesem Versuch zeigten die Wirkstoffe 2, 3, 4 und 5 bei Anwendung als 0,025 ; 0,012 oder 0,06 %ige (Gew.%) Spritzbrühen eine bessere fungizide Wirkung als das bekannte Vergleichsmittel A.

## Versuch 2

Wirksamkeit gegen Erysiphe cichoracearum an Gurken

Blätter von in Töpfen gewachsenen Gurkenkeimlingen werden mit wäßrigen Emulsionen aus 80 % Wirkstoff und 20 % Emulgiermittel (bezogen auf die Trockensubstanz) besprüht und nach dem Antrocknen des Spritzbelages mit Oidien (Sporen) des Gurkenmehltaus (Erysiphe cichoracearum) bestäubt. Die Versuchspflanzen werden anschließend im Gewächshaus bei Temperaturen zwischen 20 und 22 °C und 75 bis 80 % relativer Luftfeuchtigkeit aufgestellt. Nach 10 Tagen wird das Ausmaß der Mehltaupilzentwicklung ermittelt.

In diesem Versuch zeigten die Wirkstoffe 3, 4 und 5 bei Anwendung als 0,025 %ige Spritzbrühe eine bessere fungizide Wirkung als der bekannte Vergleichswirkstoff A.

Versuch 3

Wirksamkeit gegen Phytophthora infestans an Tomaten

Blätter von Tomatenpflanzen der Sorte « Professor Rudloff » werden mit wäßrigen Suspensionen, die 80 % (Gewichtsprozent) des zu prüfenden Wirkstoffes und 20 % Natriumligninsulfonat in der Trockensubstanz enthalten, besprüht. Es werden 0,012- und 0,006 %ige Spritzbrühen verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann :

In diesem Versuch zeigten die Wirkstoffe 3 und 5 eine bessere fungizide Wirkung als der bekannte Vergleichswirkstoff B.

**Ansprüche** (für die Vertragsstaaten : BE, CH, DE, FR, GB, IT, LI, LU, NL, SE)

1. Keten-O,N-acetal der allgemeinen Formel I

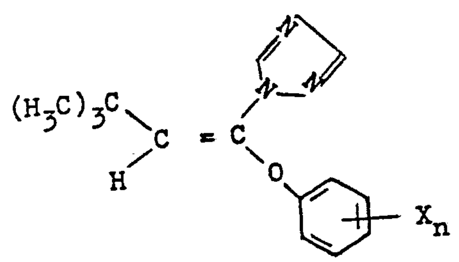

(I)

in der X Halogen, Alkyl mit 1 bis 4 C-Atomen oder Phenyl bedeutet und n eine ganze Zahl von 1 bis 3 bedeutet.

2. Fungizid enthaltend ein Keten-O,N-acetal gemäß Anspruch 1.

3. Fungizid enthaltend einen festen oder flüssigen Trägerstoff und ein Keten-O,N-acetal gemäß Anspruch 1.

4. Verfahren zur Herstellung eines Fungizids, dadurch gekennzeichnet, daß man einen festen oder flüssigen Trägerstoff mit einem Keten-O,N-acetal gemäß Anspruch 1 vermischt.

5. Verfahren zur Bekämpfung von Pilzen, dadurch gekennzeichnet, daß man die Pilze oder die vor Pilzbefall zu schützenden Gegenstände mit einem Keten-O,N-acetal gemäß Anspruch 1 behandelt.

6. Verfahren zur Herstellung eines Keten-O,N-acetals gemäß Anspruch 1, dadurch gekennzeichnet, daß man ein Sulfonat der allgemeinen Formel II

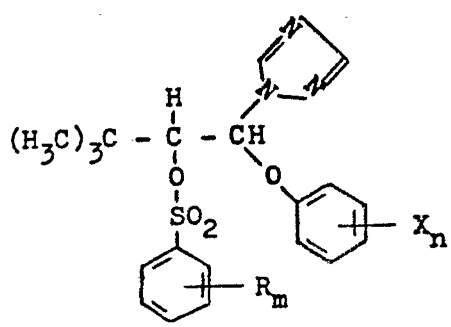

(II)

worin X und n die im Anspruch 1 angegebene Bedeutung haben und R Wasserstoff, Halogen, Alkyl mit 1

bis 4 C-Atomen oder Alkoxy mit 1 bis 4 C-Atomen und m 1 bis 3 bedeutet, in Gegenwart eines Lösungsmittels und eines Katalysators bei Temperaturen zwischen 10 und 100 °C zersetzt.

7. 1-(1,2,4-Triazol-1-yl)-1-(2,4-dichlorphenoxy)-3,3-dimethyl-buten-1.

**Ansprüche** (für den Vertragsstaat AT)

1. Fungizid enthaltend ein Keten-O,N-acetal der allgemeinen Formel I

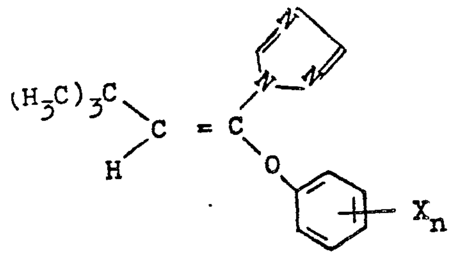

(I)

in der X Halogen, Alkyl mit 1 bis 4 C-Atomen oder Phenyl bedeutet und n eine ganze Zahl von 1 bis 3 bedeutet.

2. Fungizid enthaltend einen festen oder flüssigen Trägerstoff und ein Keten-O,N-acetal gemäß Anspruch 1.

3. Verfahren zur Herstellung eines Fungizids, dadurch gekennzeichnet, daß man einen festen oder flüssigen Trägerstoff mit einem Keten-O,N-acetal gemäß Anspruch 1 vermischt.

4. Verfahren zur Bekämpfung von Pilzen, dadurch gekennzeichnet, daß man die Pilze oder die vor Pilzbefall zu schützenden Gegenstände mit einem Keten-O,N-acetal gemäß Anspruch 1 behandelt.

5. Verfahren zur Herstellung eines Keten-O,N-acetals gemäß Anspruch 1, dadurch gekennzeichnet, daß man ein Sulfonat der allgemeinen Formel II

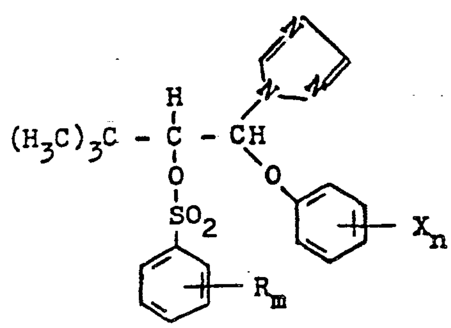

(II)

worin X und die im Anspruch 1 angegebene Bedeutung haben und R Wasserstoff, Halogen, Alkyl mit 1 bis 4 C-Atomen oder Alkoxy mit 1 bis 4 C-Atomen und m 1 bis 3 bedeutet, in Gegenwart eines Lösungsmittels und eines Katalysators bei Temperaturen zwischen 10 und 100 °C zersetzt.

6. Fungizid gemäß Anspruch 1 enthaltend 1-(1,2,4-Triazol-1-yl)-1-(2,4-dichlorphenoxy)-3,3-dimethyl-buten-1.

**Claims** (for the Contracting States : BE, CH, DE, FR, GB, IT, LI, LU, NL, SE)

1. A ketene O,N-acetal of the general formula I

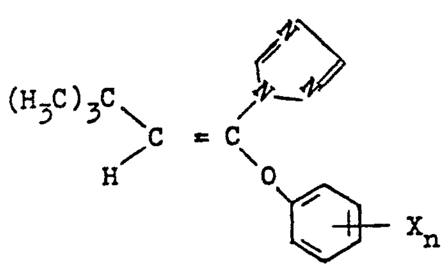

(I)

where X is halogen, alkyl of 1 to 4 carbon atoms or phenyl, and n is an integer from 1 to 3.

2. A fungicide containing a ketene 0,N-acetal as claimed in claim 1.

3. A fungicide containing a solid or liquid carrier and a ketene O,N-acetal as claimed in claim 1.

4. A process for producing a fungicide, wherein a solid or liquid carrier is mixed with a ketene O,N-acetal as claimed in claim 1.

5. A process for combating fungi, wherein the fungi or the objects to be protected against fungus attack are treated with a ketene O,N-acetal as claimed in claim 1.

6. A process for preparing a ketene O,N-acetal as claimed in claim 1, wherein a sulfonate of the general formula II

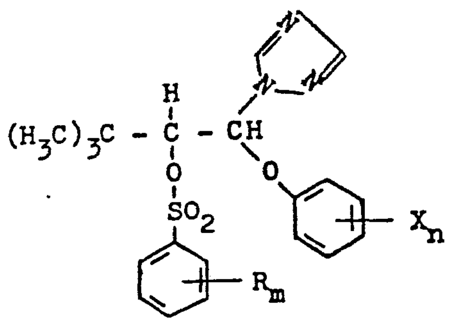

(II)

where X and n have the meanings given in claim 1, R is hydrogen, halogen, alkyl of 1 to 4 carbon atoms or alkoxy of 1 to 4 carbon atoms and m is from 1 to 3, is decomposed, at from 10 to 100 °C, in the presence of a solvent and a catalyst.

7. 1-(1,2,4-Triazolyl-1-yl)-(2,4-dichlorophenoxy)-3,3-dimethyl-butene-1.

**Claims** (for the Contracting State AT)

1. A fungicide containing a ketene O,N-acetal of the general formula I

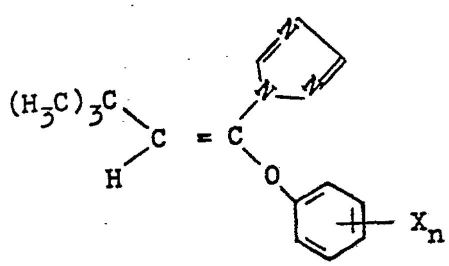

(I)

where X is halogen, alkyl of 1 to 4 carbon atoms or phenyl, and n is an integer from 1 to 3.

2. A fungicide containing a solid or liquid carrier and a ketene O,N-acetal as defined in claim 1.

3. A process for producing a fungicide, wherein a solid or liquid carrier is mixed with a ketene O,N-acetal as defined in claim 1.

4. A process for combating fungi, wherein the fungi or the objects to be protected against fungus attack are treated with a ketene O,N-acetal as defined in claim 1.

5. A process for preparing a ketene O,N-acetal as defined in claim 1, wherein a sulfonate of the general formula II

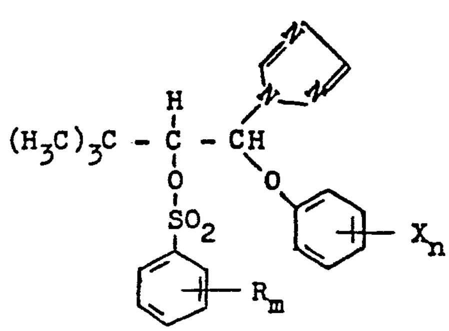

(II)

where X and n have the meanings given in claim 1, R is hydrogen, halogen, alkyl of 1 to 4 carbon atoms or

alkoxy of 1 to 4 carbon atoms and m is from 1 to 3, is decomposed, at from 10 to 100 °C, in the presence of a solvent and a catalyst.

6. A fungicide as claimed in claim 1, containing 1-(1,2,4-triazolyl-1-yl)-1-(2,4-dichlorophenoxy)-3-3-dimethyl-butene-1.

**Revendications** (pour les Etats contractants : BE, CH, DE, FR, GB, IT, LI, LU, NL, SE)

1. Cétène-O,N-acétal de la formule générale I

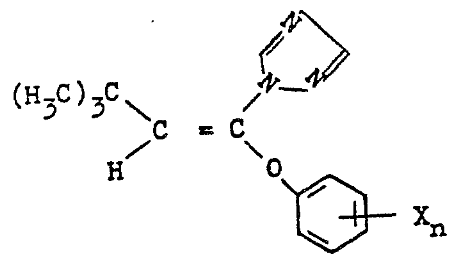

(I)

dans laquelle X désigne un halogène, un radical alkyle en $C_1$ à $C_4$ ou un groupe phényle et n est un nombre entier de 1 à 3.

2. Fongicide, contenant un cétène-O,N-acétal selon la revendication 1.

3. Composition fongicide, contenant un véhicule solide ou liquide et un cétène-O,N-acétal selon la revendication 1.

4. Procédé de préparation d'une composition fongicide, caractérisé en ce que l'on mélange un cétène-O,N-acétal selon la revendication 1 et un véhicule liquide ou solide.

5. Procédé de lutte contre des champignons, caractérisé en ce que l'on traite les champignons ou les objets à protéger des champignons avec un cétène-O,N-acétal selon la revendication 1.

6. Procédé de préparation d'un cétène-O,N-acétal selon la revendication 1, caractérisé en ce que l'on décompose un sulfonate de la formule générale II.

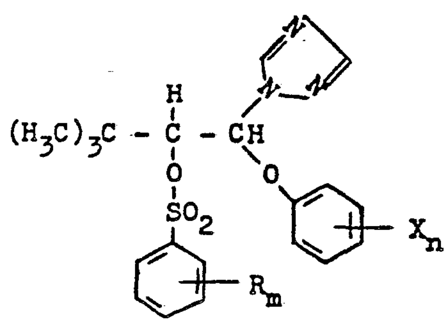

(II)

dans laquelle X et n possèdent les significations définies dans la revendication 1, R représente un atome d'hydrogène ou d'halogène ou un radical alkyle en $C_1$ à $C_4$ ou alcoxy en $C_1$ à $C_4$ et m vaut 1 à 3, en présence d'un solvant et d'un catalyseur à des températures comprises entre 10 et 100 °C.

7. Le 1-(1,2,4-triazol-1-yl)-1-(2,4-dichlorophénoxy)-3-3-diméthyl-butène-1.

**Revendications** (pour l'Etat contractant AT)

1. Fongicide, contenant un cétène-O,N-acétal de la formule générale I

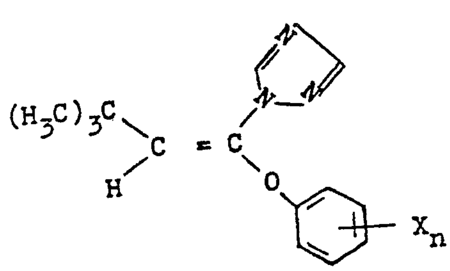

(I)

dans laquelle X désigne un halogène, un radical alkyle en $C_1$ à $C_4$ ou un groupe phényle et n est un nombre entier de 1 à 3.

2. Fongicide, contenant un véhicule solide ou liquide et un cétène-O,N-acétal selon la revendication 1.

3. Procédé de préparation d'un fongicide, caractérisé en ce que l'on mélange un cétène-O,N-acétal selon la revendication 1 avec un véhicule solide ou liquide.

4. Procédé de lutte contre des champignons, caractérisé en ce que l'on traite les champignons ou les objets à protéger des champignons avec un cétène-O,N-acétal selon la revendication 1.

5. Procédé de préparation d'un cétène-O,N-acétal selon la revendication 1, caractérisé en ce que l'on décompose un sulfonate de la formule générale II

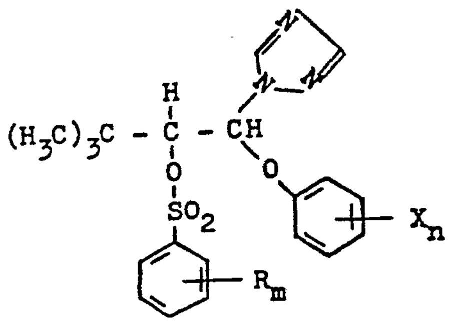

(II)

dans laquelle X et n possèdent les significations définies dans la revendication 1, R représente un atome d'hydrogène ou d'halogène ou un radical alkyle en $C_1$ à $C_4$ ou alcoxy en $C_1$ à $C_4$ et m vaut 1 à 3, en présence d'un solvant et d'un catalyseur à des températures comprises entre 10 et 100 °C.

6. Le 1-(1,2,4-triazol-1-yl)-1-(2,4-dichlorophénoxy)-3,3-diméthyl-butène-1.